# EUROPÄISCHE PATENTANMELDUNG

(11) **EP 2 626 005 A1**
(43) Veröffentlichungstag der Anmeldung: **14.08.2013**
(21) Anmeldenummer: 12154764.0
(22) Anmeldetag: 09.02.2012
(51) Int. Cl.: A61B 5/024, A61B 5/16, A61B 5/0456

(54) **Verfahren zur Bestimmung des physischen und/oder psychischen Zustands eines Probanden anhand einer Analyse seiner Herzratenvariabilität**

(30) Priorität: 08.02.2012 EP 12154578
(71) Anmelder: PULSE7 GmbH, 4614 Marchtrenk (AT)
(72) Erfinder: PULSE7 GmbH, 4614 Marchtrenk (AT)
(74) Vertreter: Jell, Friedrich

(57) **Zusammenfassung**

Es wird ein Verfahren zur Bestimmung des physischen und/oder psychischen Zustands eines Probanden anhand einer Analyse seiner Herzratenvariabilität (HRV) gezeigt, bei welcher Analyse wenigstens eine Häufigkeitsverteilung von in mindestens einem Untersuchungszeitraum aufgenommenen Interbeat-Intervallen (IBI) untersucht wird. Um hierfür eine besonders aussagekräftige und schnell zu interpretierende Informationen zur Herztätigkeit zu erhalten, wird vorgeschlagen, dass die Analyse der Herzratenvariabilität (HRV) mindestens eine Untersuchung wenigstens einer Häufigkeitsverteilung auf eine multimodale Verteilung umfasst.

## Beschreibung

Die Erfindung betrifft ein Verfahren zur Bestimmung des physischen und/oder psychischen Zustands eines Probanden anhand einer Analyse seiner Herzratenvariabilität (HRV), bei welcher Analyse wenigstens eine Häufigkeitsverteilung von in mindestens einem Untersuchungszeitraum aufgenommenen Interbeat-Intervallen (IBI) untersucht wird.

Um den physischen und/oder psychischen Zustand eines Probanden zu bestimmen, ist aus dem Stand der Technik bekannt, den Ruhepuls bzw. die Pulsfrequenz - evtl. bei unterschiedlichen Atemvorgaben - zu eruieren und die dabei erhaltenen Ergebnisse insbesondere in Relation zu Vergleichswerten zu setzten. Derartige Verfahren können eventuell Richtwerte hinsichtlich des Zustands bzw. der Leistungsfähigkeit eines Probanden liefern, allerdings erweisen sie sich als vergleichsweise aufwendig, weil die Bedingungen, unter denen die Bestimmung durchgeführt werden, nur schwer standardisierbar sind. Zudem sind die Ergebnisse derartige Verfahren auch nur bedingt aussagekräftig, nicht zuletzt deshalb, weil eine mittlere Herzfrequenz bestimmt wird, diese jedoch nur ein Ergebnis der ihr zugrundeliegenden Herzschläge darstellt.

Des Weiteren ist bekannt, über einen bestimmten Zeitraum Interbeat-Intervalle (IBI), bzw. insbesondere RR-Intervalle der Herztätigkeit zu bestimmen und über die Quantifizierung der dabei gemessenen Schwankungen eine Herzratenvariabilität zu berechnen, um so auf den Einfluss des vegetativen Nervensystems auf die Herztätigkeit und in weiterer Folge Rückschlüsse auf den psychischen und physischen Zustand ziehen zu können. Bekannt ist nämlich, dass sich zwei Personen, die den gleichen Herzrhythmus aufweisen, in ihrem Regulationszustand durch das vegetative Nervensystem und somit in ihrem Gesundheitszustand und Leistungsfähigkeit erheblich unterscheiden können. Üblicherweise wird bei derartigen Verfahren versucht, die Häufigkeit von Ergebnissen einer IBI-Messung in Form einer Gauß-Kurve darzustellen. Aus der Lage dieses Maximums bzw. der Gauß-Kurve, der Verteilung der, dieses Maximum umgebenden Klassen bzw. der Standardabweichung und/oder Symmetrie der Gauß-Kurve etc. werden Rückschlüsse auf den physischen und/oder psychischen Zustand eines Probanden gezogen. Allerdings erweist sich ein derartiges Verfahren als aufwendig, denn es ist meist sogar erforderlich, die Dauer der Messung zur gewünschten Klassierung eines Maximums vergleichsweise lange, beispielsweise 30 Minuten zu wählen. Zudem ist ein Verfahren nach dem Stand der Technik auch nur bedingt aussagekräftig. Unter anderem findet zwar Berücksichtigung, dass die Herztätigkeit je nach Zustand des Probanden durch unterschiedlich variable RR-Intervall-Längen gekennzeichnet ist, allerdings wird für deren Beurteilung im Wesentlichen von einer, durch einen einzigen Modus bestimmten Grundfrequenz ausgegangen.

Die Erfindung hat sich daher die Aufgabe gestellt, ein Verfahren zu entwickeln, mit dessen Hilfe auf den physischen und/oder psychischen Zustand eines Probanden einfach, rasch und reproduzierbar geschlossen werden kann. Zudem sollte das erfindungsgemäße Verfahren weitestgehend unabhängig von Parametern wie Umgebungstemperatur, Tageszeit etc. angewendet werden können.

Die Erfindung löst die Aufgabe dadurch, dass die Analyse der Herzratenvariabilität (HRV) mindestens eine Untersuchung wenigstens einer Häufigkeitsverteilung auf eine multimodale Verteilung umfasst.

Umfasst die Analyse der Herzratenvariabilität (HRV) mindestens eine Untersuchung wenigstens einer Häufigkeitsverteilung auf eine multimodale Verteilung, können besonders aussagekräftige Informationen zur Herztätigkeit erhalten werden, auf deren Grundlage ein Rückschluss auf den psychischen und/oder physischen Zustand eines Probanden möglich ist. Es kann nämlich davon ausgegangen werden, dass ein Auftreten mehrerer Modalwerte, ein Hinweis für einen besonders nachteiligen Regulationszustand des Herzens durch das vegetative Nervensystem sein kann, und zwar selbst dann, wenn zwei Modalwerte mit sehr geringer Differenz hinsichtlich der Längen ihrer Interbeat-Intervalle (IBI) identifizierbar sind. Dabei spielen auch nicht unbedingt Ausprägung, Lage etc. eines oder mehrerer zusätzlicher Modalwerte eine Rolle, alleine die Tatsache, dass mehr als ein Modalwert identifiziert werden kann, kann wichtige Informationen liefern und sogar als grundlegender Indikator für eine, von einem wünschenswerten Regulationszustand des Herzens abweichende Herztätigkeit herangezogen werden. Mehr als ein Modalwert kann nämlich als eine Art Abweichung von einer Grundfrequenz ausgelegt werden. Es kann also bedeuten, dass die Herztätigkeit nicht nur durch eine Variabilität der IBI-Längen um eine sogenannte Grundschwingung bestimmt wird, welche Grundschwingung durch eine bestimmte Bandbreite an IBI-Längen um ein Maximum, das durch besonders viele gemessene Interbeat-Intervall-Längen gekennzeichnet ist. Vielmehr bildet die Herztätigkeit also mehrere Maxima an Häufigkeiten von Interbeat-Intervallen aus, die vom vegetativen Nervensystem hinsichtlich ihrer Längen beeinflusst werden.

Werden als Interbeat-Intervalle RR-Intervalle des Herzens und/oder Abstände zwischen Pulswellen herangezogen, kann eine besonders genaue bzw. einfache Bestimmung der Herzratenvariabilität erfolgen. Der Vollständigkeit halber wird erwähnt, dass RR-Intervall den Abstand zwischen zwei R-Zacken eines Elektrokardiogramms bezeichnet, selbstverständlich können sich aber auch Punkte und/oder Ableitungen eines EKGs für das erfindungsgemäße Verfahren eignen. Werden die Abstände zwischen Pulswellen herangezogen, kann eine besonders einfache und trotzdem genaue Bestimmung ermöglicht werden. So kann beispielsweise eine Bestimmung von Inter-Beat-Intervallen mithilfe bekannter entsprechender Vorrichtungen - exemplarisch erwähnt seien Ohrclip oder Fingersensor, die etwa mit Infrarot die Änderung der Lichtdurchlässigkeit der Haut messen oder auch Handpulssensoren. Hier bietet sich beispielsweise an, das errechnete Maximum der Pulswelle oder auch den Beginn eines bestimmten Anstiegs zu bestimmten.

Erfolgt die Untersuchung der Häufigkeitsverteilung auf einer univariaten statistischen Analyse, ist auf schnelle und einfache Weise eine aussagekräftige Analyse der Herzratenvariabilität möglich. Als Beispiel sei hierzu ein Histogramm erwähnt, in dem die Längen der in einem Untersuchungszeitraum aufgenommenen IBI nach ihrer Häufigkeit eingetragen werden.

Wird eine klassierte Häufigkeitsverteilung herangezogen, kann eine vereinfachte Darstellung, Analyse und/oder Verarbeitung der erhaltenen Messwerte erfolgen. Zu beachten ist selbstverständlich, dass die Klassengrenzen bzw. die Klassenbreiten derart festgesetzt werden, dass sich eventuelle Modalwerte nicht überschneiden, überlagern etc. und das erfindungsgemäße Verfahren anderwärtig negativ beeinträchtigen. Selbstverständlich kann dabei auch eine Rolle spielen, welche Länge des Untersuchungszeitraums für die Messung von Interbeat-Intervallen gewählt wird. Eine Langzeitmessung von 20 Minuten und mehr, wie dies im Stand der Technik üblicherweise vorkommt, kann eine Überlagerung eventueller Modi klarerweise begünstigen oder die Analyse zumindest erheblich erschweren. Vorteilhaft berücksichtigt das erfindungsgemäße Verfahren also sowohl die Anzahl an identifizierten Maxima, als auch eine Variabilität der IBI um diese Maxima. Um derartige Modi zu identifizieren sind verschiedenste Verfahren bekannt, unter anderem Schwellwertanalysen.

Erfolgt die Untersuchung der Häufigkeitsverteilung auf einer bivariaten statistischen Analyse, kann sich so unter anderem eine präzisere Analyse der HRV ergeben, da insbesondere Zusammenhänge von Inter-Beat-Intervallen dargestellt werden können. Werden mehr als eine Ausprägung zur Analyse herangezogen, kann also ein verbesserter Rückschluss auf die Herztätigkeit erfolgen.

Wird für die bivariate statistische Analyse mindestens ein Interbeat-Intervall mit seinem nachfolgenden Interbeat-Intervall herangezogen, kann eine besonders genaue Analyse der HRV ermöglicht werden, da unter anderem erkennbar sein kann, wie sprunghaft sich die Längen aufeinanderfolgender Inter-Beat-Intervalle ändern. Starke Schwankungen der Längen nacheinander auftretender Interbeat-Intervalle könnten also die Annahme nahelegen, dass sich der Regulationszustand des Herzens in einem vergleichsweise unausgewogenen Zustand befindet, was wiederum entsprechende Rückschlüsse auf den physischen und/oder psychischen Zustand eines Probanden zulassen kann. Bilden Interbeat-Intervalle, deren jeweils nachfolgendes IBI sich in seiner Länge wesentlich unterscheidet, sogar einen Modus oder mehrere Modi aus, kann somit von einem vergleichsweise nachteiligen Regulationszustand der Herztätigkeit ausgegangen werden.

Umfasst die bivariate statistische Analyse eine Klassierung der Häufigkeitsverteilung in Abhängigkeit der Unterschiede zweier aufeinanderfolgender Interbeat-Intervall-Längen, kann eine aussagekräftige und vor allem auch leicht zu interpretierende Informationen über die HRV bzw. die Aktivität des vegetativen Nervensystems eines Probanden erhalten werden. Wiederum können auf Grundlage einer derartigen Analyse verbessert Rückschlüsse auf psychischen und/oder physischen Zustand eines Probanden liefern, auf deren Grundlage beispielsweise Gesundheitszustand, Regenerationsfähigkeit, ideale Intensität einer körperlichen Trainingseinheit und so weiter verbessert beurteilt werden können.

Berücksichtigt die Analyse der Herzratenvariabilität den Abstand zwischen Modalwerten, kann die Aussagekraft des erfindungsgemäßen Verfahrens weiter verbessert werden. Eine große Differenz zweier eventuell festgestellter Modalwerte hinsichtlich der Längen der IBI-Intervalle kann entsprechend obenstehender Ausführung den Schluss auf eine vergleichsweise nachteilige Herzratenvariabilität nahelegen - und zwar umso nachteiliger, je größer diese ermittelte Differenz erscheint.

Zur weiteren Verbesserung der Beurteilung der Herzratenvariabilität kann die Analyse der Herzratenvariabilität ein Verhältnis der Dichten der Modalverteilungen, insbesondere der Integrale dieser Dichten, berücksichtigen. Somit können erleichtert etwa Verhältnisse der Modi zueinander bzw. auch die Mengenanteile der, den jeweiligen Modi zugehörigen RR-Intervalle dargestellt bzw. ausgewertet werden. Dies kann sich auch für Streudiagramme mit aufeinanderfolgenden Interbeat-Intervallen eignen, wobei die Dauer jedes Interbeat-Intervalls eines bestimmten Messzeitraums in Abhängigkeit der Dauer des diesem nachfolgenden Interbeat-Intervalls eingetragen wird. Eine graphische Darstellung könnte hierbei ein zweidimensionales Bild ergeben, in dem die Dichte der Modalverteilung farblich oder durch Höhenlinien gekennzeichnet wird - selbstverständlich wären aber auch andere Darstellungen des erfindungsgemäßen Verfahrens vorstellbar, etwa eine dreidimensionale. Auf diese Weise kann eine wesentlich schnellere bzw. vereinfachte Analyse der HRV ermöglicht werden und zwar insbesondere auch unter Berücksichtigung der Ausprägung mehrerer Häufigkeiten, beziehungsweise deren Verhältnisse, Relation und/oder Abhängigkeit zueinander.

Die erfindungsgemäße Analyse kann weiter verbessert werden, wenn bei der Bestimmung des physischen und/oder psychischen Zustands eines Probanden die Analysedaten der Herzratenvariabilität (HRV) mit Vergleichsdaten anderen Probanden in Relation gesetzt werden. Es sei erwähnt, dass es hierbei von besonderem Vorteil sein kann, zur Heranziehung entsprechender Vergleichsdaten eine Erhebung der Lebensumstände des Probanden durchzuführen. Auf diese Weise können nicht nur Beurteilungen analysierter HRV erleichtert erfolgen, sondern auch eventuell erforderliche Maßnahmen zur Verbesserung einer HRV vereinfacht festgelegt und/oder überprüft werden. Zudem ist es selbstverständlich vorstellbar, Analysen verschiedener Probanden oder Probandengruppen nach bestimmten Kriterien heranzuziehen, um Modelle zu entwickeln, um so wiederum Rückschlüsse auf den physischen und/oder psychischen Zustand eines Probanden durchzuführen.

Werden die Modalwerte der Häufigkeitsverteilung unter Verwendung eines Schwellwerts bestimmt, kann beispielsweise ermöglicht werden, eine Klassierung einer multimodalen Verteilung erleichtert vorzunehmen und/oder besonders deutlich darzustellen. Dies kann auch den Vorteil haben, dass die, nicht zuletzt aufgrund einer im Stand der Technik üblichen Messdauer von 20 Minuten und mehr, als vermeintliche statistische Ausreißer erscheinenden Messergebnisse ebenfalls erleichtert Berücksichtigung finden können.

Besonders aufschlussreich kann sich das erfindungsgemäße Verfahren darstellen, wenn die Häufigkeitsverteilungen von in wenigstens zwei Untersuchungszeiträumen aufgenommenen Interbeat-Intervallen untersucht werden, wobei in den Untersuchungszeiträumen unterschiedliche physische und/oder psychische Belastungszustände auf den Probanden wirken. Auf diese Weise kann beispielsweise die Anpassungsfähigkeit eines Probanden an unterschiedliche Belastungen ermittelt werden. Es kann etwa auch vereinfacht festgestellt werden, welche Belastungsintensität - etwa für körperliches Training - den Erfordernissen oder dem Zustand des Probanden am besten entspricht. Auch die Wirkung eines eventuellen Entspannungstrainings, einer Umstellung von Lebensgewohnheiten kann dadurch schnell und einfach festgestellt und/oder optimiert werden. Es ist dazu lediglich erforderlich, Veränderungen der Herzratenvariabilität und insbesondere auch eventuell ausgebildeter Maxima in regelmäßigen Zeitabständen zu prüfen. Vielfach kann beispielsweise davon ausgegangen werden, dass eine Verringerung des Abstands zwischen ermittelten Modalwerten als positive Entwicklung positiv zu bewerten sind. Erfindungsgemäß kann für eine aussagekräftige Analyse eine vergleichsweise kurze Messdauer der Herztätigkeit im Bereich weniger Minuten ausreichen.

Vorteilhaft kann mit Hilfe der Untersuchung der Häufigkeitsverteilung ein Korrekturwert für eine Zeitbereichsanalyse der Herzfrequenzvariabilität, beispielsweise für Parameter RMSSD und/oder pNN50, berechnet und dabei berücksichtigt werden.

Es sei erwähnt, dass unter Proband selbstverständlich eine Person zu verstehen ist, unabhängig von Alter, Geschlecht, Gesundheitszustand oder anderen Merkmalen.

In den Zeichnungen wird der Erfindungsgegenstand beispielsweise anhand von Figuren näher erläutert. Es zeigen
- Fig. 1: eine Darstellung von in einem bestimmten Untersuchungszeitraum ermittelten RR Intervall-Längen,
- Fig. 2: ein Histogramm der RR-Intervalle nach den Ergebnissen in Fig. 1,
- Fig. 3: die RR Intervalle der Ergebnisse in Fig. 1 als Streudiagramm,
- Fig. 4 bis 6: ein Beispiel einer anderen RR-Intervallbestimmung entsprechend den Darstellungen nach Fig. 1 bis 3,
- Fig. 7: eine weitere graphische Darstellung eines anderen Messergebnisses samt einem Histogramm von RR-Intervallpaaren, aufgetragen in Abhängigkeit der Differenz ihrer Längen zueinander.

Der Vollständigkeit halber wird erwähnt, dass in den Figuren RR-Intervalle untersucht und dargestellt werden, es aber selbstverständlich im Rahmen der Erfindung liegt, auch andere Parameter und/oder Messmethoden zur Ermittlung von Interbeat-Intervallen heranzuziehen.

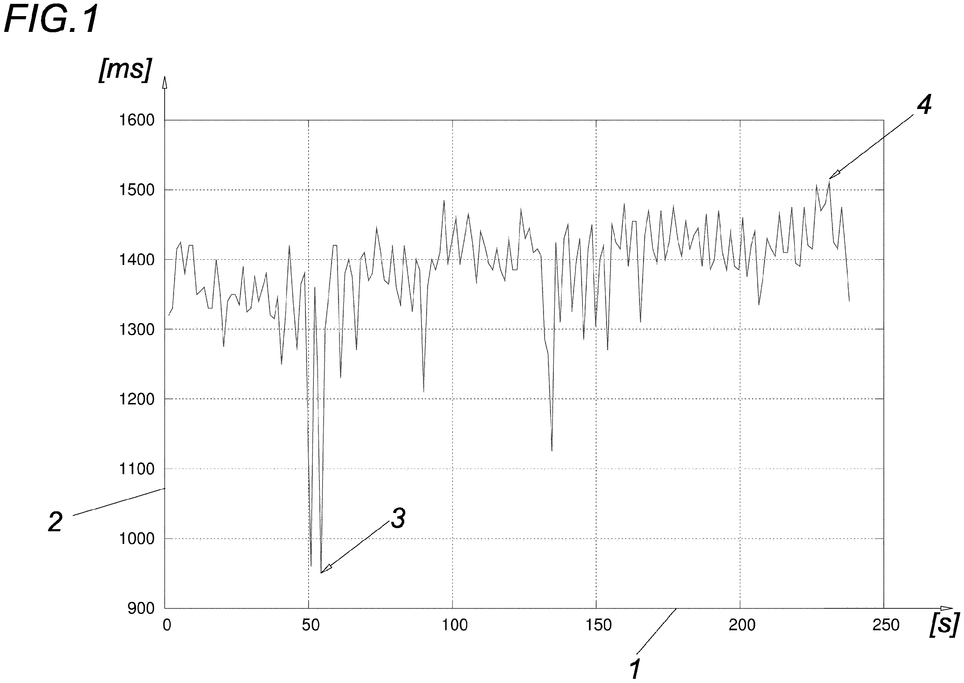

Gemäß Fig. 1 werden beispielhaft die in einem Untersuchungszeitraum 1 aufgenommenen RR-Intervalle aufeinanderfolgender Herzschläge eines Probanden graphisch dargestellt. Dabei werden die entlang der x-Achse des Untersuchungszeitraums 1 in Sekunden [s] auftretenden Zeitlängen 2 der RR-Intervalle in Millisekunden [ms] aufgetragen und aufeinanderfolgende RR-Intervalle durch eine gerade Linie miteinander verbunden. Beispielsweise können der Darstellung nach Fig. 1 so das minimale und maximale RR-Intervall 3 und 4 eines bestimmten Zeitraums entnommen werden. Eine andere Darstellung, beispielsweise als RR-Tachogramm ist ebenso vorstellbar, jedoch nicht dargestellt.

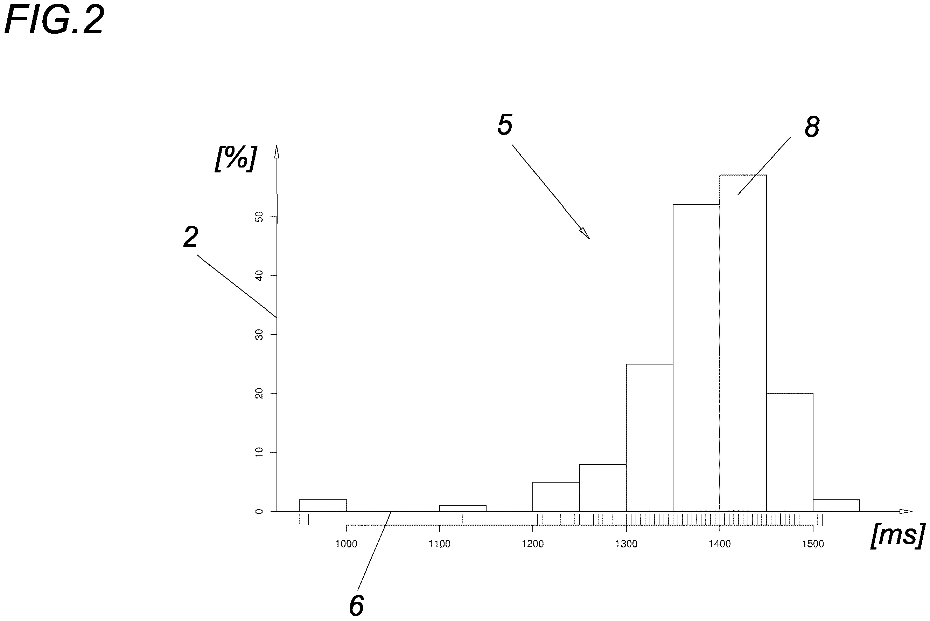

Ebenso kann aus den in Figur 1 dargestellten Werten eine Häufigkeitsverteilung nach Fig. 2 abgeleitet werden. Fig. 2 stellt eine univariate statistische Analyse in Form eines Histogramms 5 dar, in welchem beispielsweise alle in einem bestimmten Zeitraum ermittelten RR-Intervalle in 50ms-Klassen zusammengefasst werden, wobei die x-Achse 6 die Länge von RR-Intervallen in ms kennzeichnet. Die Höhen dieser als Balken dargestellten Klassen ergeben sich in diesem Histogramm entsprechend der Skalierung der y-Achse 7 und der, der jeweiligen Klasse zugehörigen Anzahl an RR-Intervallen. In diesem Beispiel ist ein Modalwert 8 zu erkennen. Aus Anzahl und Höhe der Klassen kann die Schwankungsbreite der Länge der RR-Intervalle - beispielsweise auch durch Beurteilung gegenüber Vergleichswerten - abgeleitet werden. Ein derartiges Histogramm kann noch bearbeitet werden, etwa um das Verhältnis der Dichten der Modalverteilungen, insbesondere der Integrale dieser Dichten, zu berücksichtigen, was in Fig. 2 nicht näher dargestellt wird. Zeigt die Häufigkeitsverteilung lediglich einen Modus sowie eine im Wesentlichen normale, symmetrische Dichterverteilung - ähnlich einer Gauß-Kurve -, wird entsprechend dem Stand der Technik von einer vergleichsweise guten Variabilität der Herzraten und einem guten Zustand eines Probanden gesprochen.

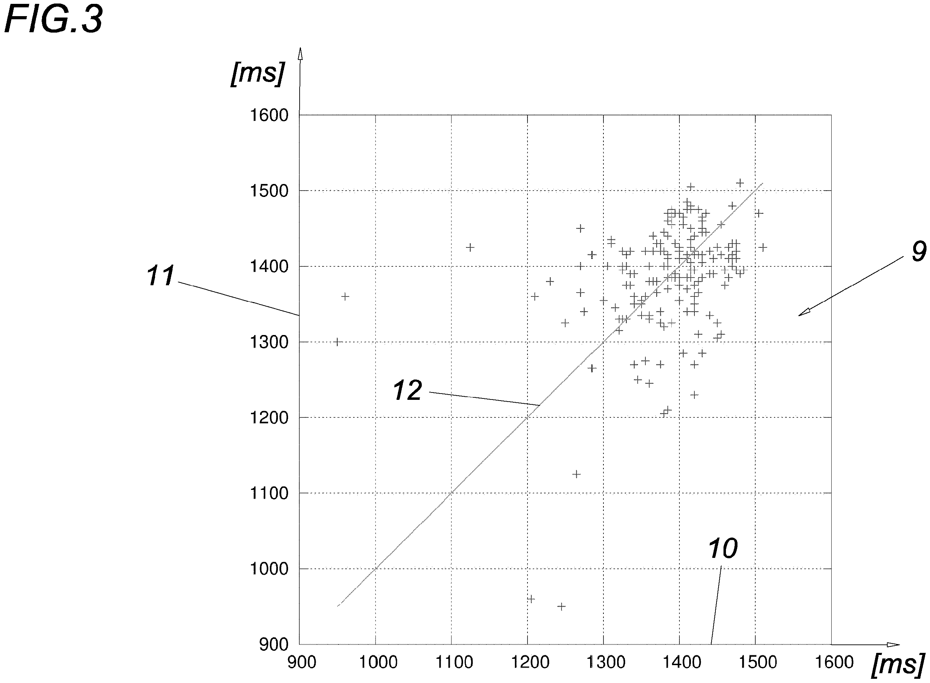

Des Weiteren kann der Informationsgehalt der Werte nach Fig. 1 in bekannter Weise auch als Streudiagramm (Scatterplot) entsprechend der Fig. 3 wiedergegeben werden. Eine derartige bivariate statistische Analyse kann beispielsweise auf Grundlage zweier aufeinanderfolgender RR-Intervalle erfolgen. Dabei wird die Dauer jedes Herzschlags 10 in ms auf der x-Achse in Abhängigkeit der Dauer des diesem Herzschlag nachfolgenden Herzschlags in ms auf der y-Achse 11 eingetragen. Es wird von einer vorteilhaften Regulationsbreite, also von einer vergleichsweise guten Herzratenvariabilität, gesprochen, wenn die eingezeichneten Punkte in diesem Diagramm eine im Wesentlichen ellipsenförmige Punktwolke ergeben, wobei die Hauptachse dieser Punktwolke im Wesentlichen über der eingezeichneten Diagonalen 12 liegt. Punkte, die auf dieser Diagonale liegen, bezeichnen zwei aufeinanderfolgende RR-Intervalle, deren Länge gleich ist.

Die mögliche Beurteilung derartiger Darstellungen nach den Figuren 1- 3 hinsichtlich des physischen und/oder psychischen Zustands eines Probanden, insbesondere auch aufgrund der Breite einer Ellipse im Streudiagramm, einer symmetrischen, links- oder rechtssteilen Gauß-Kurve, Dichterverteilung im Histogramm etc. sind im Stand der Technik ausführlich bekannt und diskutiert.

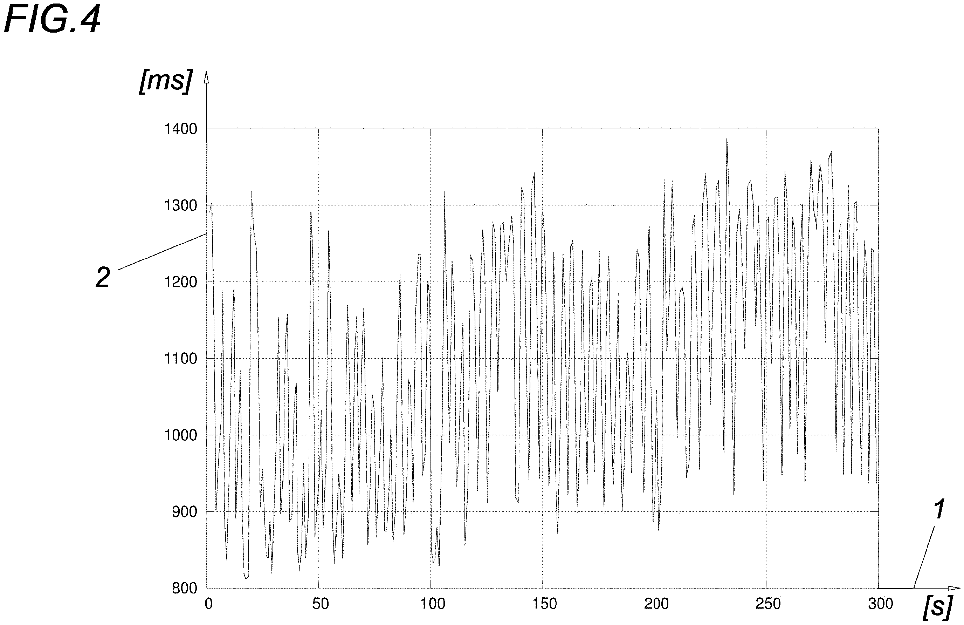

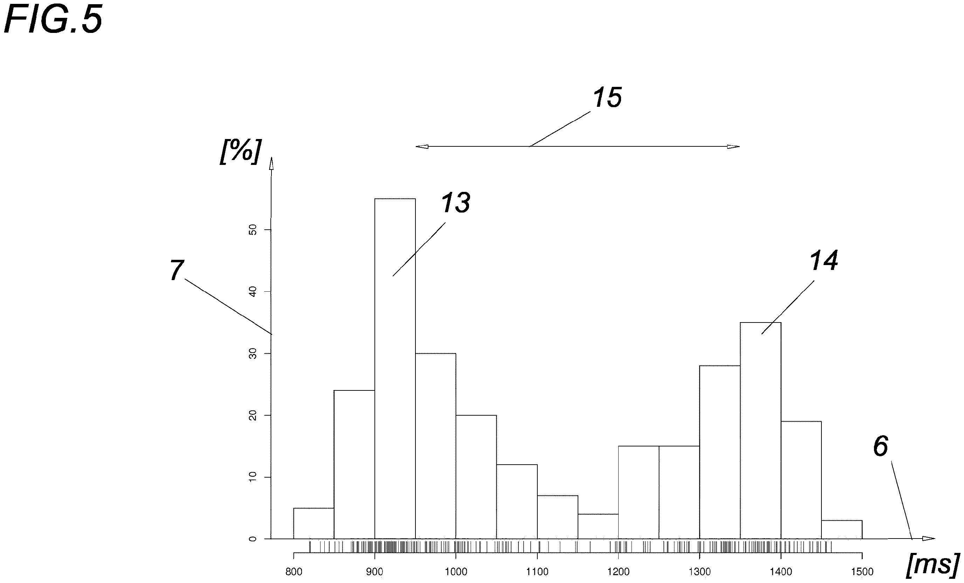

Erfindungsgemäß umfasst nun die Analyse der Herzratenvariabilität (HRV) mindestens eine Untersuchung wenigstens einer Häufigkeitsverteilung auf eine multimodale Verteilung. Um dies zu veranschaulichen wird - den vorangegangenen Figuren entsprechend - in den Figuren 4 bis 7 ein Beispiel für ein Ergebnis einer anderen RR-Intervallmessung gezeigt. In der Figur 5 ist eine multimodale Verteilung erkennbar und zwar durch die Ausbildung von zwei Maxima 13, 14, die jeweils einen Modus ausbilden. Es wird darauf hingewiesen, dass auch die Histogramme nach den Figuren 2 und 5 als beispielhafte Darstellungen zu verstehen sind und selbstverständlich von dieser abweichende Einteilungen der Klassen, Skalierungen und Einheiten der x- oder y-Achse etc. vorgenommen werden können oder müssen, um eine Durchführung des erfindungsgemäßen Verfahrens zu ermöglichen. Zur Beurteilung des Zustands eines Probanden kann beispielsweise die Beabstandung 15 ermittelter Maxima zueinander als Maß herangezogen werden. Derzeitigen Erkenntnissen entsprechend dürfte ein psychischer und/oder physischer Zustand eines Probanden umso nachteiliger eingestuft werden, je größer der Abstand zwischen zwei Modi ist. Zu berücksichtigen gilt dabei evtl. auch der Unterschied der Höhen von zwei oder mehreren ermittelten Maxima oder Modi zueinander bzw. kann auch eine Berechnung der Dichteverteilung wesentlichen Aufschluss hierzu bieten. Aus dem Beispiel der Figur 5 mit einer Häufigkeitsverteilung um zwei Maxima 13, 14 kann nämlich unter anderem abgeleitet werden, dass sich die mittlere Herzfrequenz im Wesentlichen durch zwei Grundschwingungen ausbilden könnte, wie dies bereits erläutert wurde.

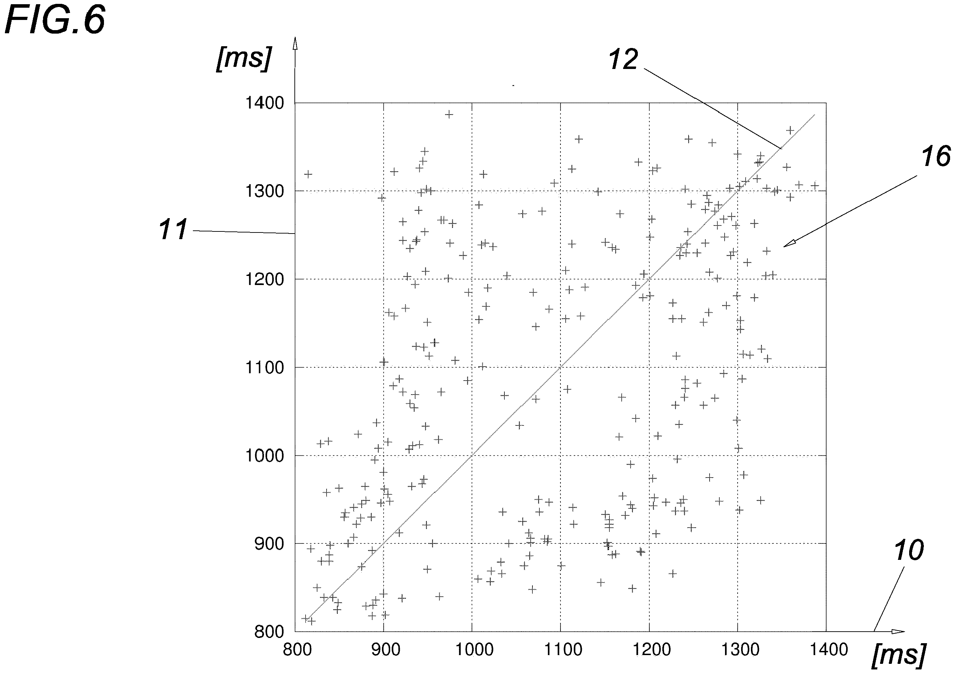

Fig. 6 zeigt wiederum ein Streudiagramm 16, anhand dessen erkennbar wird, dass eine Analyse einer Häufigkeitsverteilung vergleichsweise schwierig sein kann, da die im Streudiagramm eingetragenen Werte in der Art eines Schrotschusses erscheinen. Erkennbar ist, dass sich die Längen zweier aufeinanderfolgender Herzschläge im Wesentlichen unterscheiden - eine als im Allgemeinen als wünschenswert geltende ellipsenförmige Häufigkeitsverteilung, wie diese andeutungsweise in Fig. 3 erkennbar sein kann, ist nur schwerlich sichtbar. Es ist zudem ersichtlich, dass vergleichsweise viele RR-Intervallpaare von der Diagonalen 12 vergleichsweise weit beabstandet sind, was den Rückschluss zulassen kann, dass die Herztätigkeit unrhythmisch oder unharmonisch, also von einer Grundschwingung abweichend, erscheint.

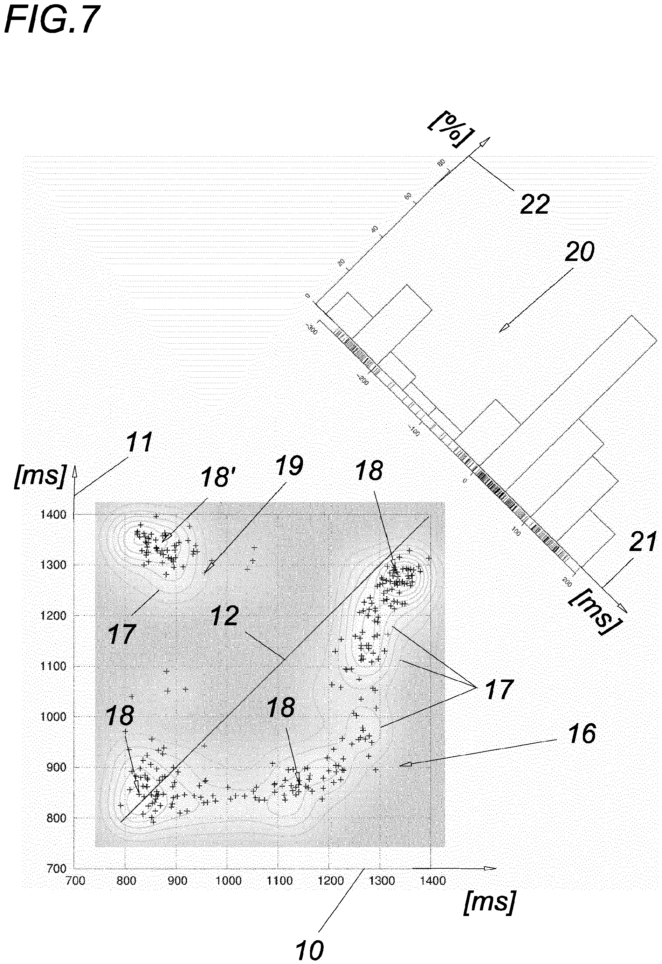

Erfindungsgemäß gilt es, das Streudiagramm auf eine multimodale Verteilung zu untersuchen. Ein mögliches Ausführungsbeispiel ist in Figur 7 dargestellt, wobei selbstverständlich auch jegliche andere Art der Darstellung von Modalwerten geeignet sein kann. In diesem Beispiel werden Modalwerte als Dichteverteilung dargestellt und deren Ausprägungen in Form von Höhenlinien 17 gekennzeichnet. Vorteilhaft kann so eine schnelle und aufschlussreiche Information zu vorhandenen Modi 18 hinsichtlich der Dauer zweier aufeinanderfolgender RR-Intervalle und/oder des Verhältnisses Ihrer RR-Intervalldauer zueinander ermöglicht werden. Auch eventuelle, lediglich als Ausreißer 19 beurteilte Werte, können auf diese Weise einfach für eine Analyse herangezogen werden bzw. in diese einfließen. Insbesondere von Vorteil zeigte sich, dass für dieses erfindungsgemäße Verfahren bereits eine Messdauer von RR-Intervallen über einen Zeitraum von wenigen Minuten ausreichen kann. Es ist also unter anderem möglich, jene Modi 18, die sich durch Häufigkeiten von RR-Intervall-Längen in Abhängigkeit der Länge ihres nachfolgenden RR-Intervalls ergeben und im Wesentlichen den Herzrhythmus während des Messintervalls bestimmen, zu identifizieren. Dementsprechend sind Rückschlüsse, beispielsweise aufgrund der Lage und/oder - je nach Darstellung - der Größe, Höhe bzw. Ausprägung einzelner Modi im Streudiagramm und/oder beispielsweise auch aufgrund des Abstands zwischen den Modi, zum psychischen und/oder physischen Zustand eines Probanden möglich. 18' zeigt beispielsweise einen Modus, der im Allgemeinen als vergleichsweise nachteilig angesehen werden kann, da dieser einander nachfolgende RR-Intervall-Längen kennzeichnet, die sich vergleichsweise stark voneinander unterscheiden bzw. sprunghafte Änderungen des Herzrhythmus kennzeichnen können. Es wird festgehalten, dass es auch vorstellbar ist, dreidimensionale Streudiagramme zu erstellen, in denen jedes Herzschlagintervall in Abhängigkeit seiner beiden nachfolgenden Herzschlagintervalle auf der y- und z-Achse aufgetragen wird.

Figur 7 zeigt zudem eine Häufigkeitsverteilung der RR-Intervallmessung als Histogramm 20, das in der Art einer Projektion des Streudiagramms 16 verstanden werden kann. Hierbei umfasst die bivariate statistische Analyse eine Klassierung der Häufigkeitsverteilung in Abhängigkeit der Unterschiede zweier aufeinanderfolgender Interbeat-Intervall-Längen. Dieses Histogramm 20 kann insbesondere eine Untersuchung auf eine multimodale Verteilung von RR-Intervallen, die sich von ihren nachfolgenden RR-Intervallen hinsichtlich ihrer Länge unterscheiden, erlauben. So werden jene aufeinanderfolgenden RR-Intervallpaare, deren Längen gleich sind, die also auf der Diagonale 12 des zugehörigen Streudiagramms liegen, der Zahl 0 der X-Achse 21 des Histogramms in Figur 7 zugeordnet. Diese X-Achse 21 kennzeichnet das Ausmaß der Differenz zweier aufeinanderfolgender RR-Intervall-Längen. Die y-Achse 22 des Histogramms gibt wiederum die Häufigkeit der RR-Intervallpaare an. Auch im Histogramm 20 der Figur 7 wird eine Untersuchung auf eine multimodale Verteilung durchgeführt, die beispielsweise Auskunft darüber geben kann, aus welchen Modi sich der Herzrhythmus über den jeweiligen Untersuchungszeitraum im Wesentlichen zusammensetzt bzw. wie sprunghaft sich aufeinanderfolgende RR-Intervalle hinsichtlich ihrer Länge unterscheiden bzw. ändern. So kann unter anderem, wenn eine multimodale Verteilung auftritt, auf eine Abweichung von einer, im Wesentlichen die mittlere Herzfrequenz bestimmenden Grundschwingung ausgegangen werden.

Vorstellbar ist auch, die Darstellung der Modalwerte zur verändern und/oder zu erleichtern. In Fig. 7 könnte sich hierbei beispielsweise anbieten, jene RR-Intervalle, die der Zahl 0 auf der X-Achse 21 entsprechen oder ihr - je nach Definition - nahe kommen, aus der graphischen Darstellung auszublenden. Somit könnte es erleichtert werden, jene Modi zu analysieren, die durch aufeinanderfolgende RR-Intervalle gebildet werden, welche sich in ihren Längen unterscheiden und eher als nachteilig anzusehen sind.

Auf diese Weise kann außerdem etwa eine Berücksichtigung der Innervation des vegetativen Nervensystems während des Messvorgangs - etwa durch körperliche Betätigung, Sinneswahrnehmung etc. des Probanden - Berücksichtigung finden. Dies kann also deshalb von Vorteil sein, weil eine derartige Innervation im Zuge einer Messung von RR-Intervallen eine Beschleunigung oder Verlangsamung des Pulses und somit eine Änderung der Längen von RR-Intervallen zur Folge haben kann. Diese Schwankungen werden jedoch, solange aufeinanderfolgende RR-Intervalle etwa gleich - oder entsprechend einer bestimmten Definition annähernd gleich - lang sind, physiologisch als vorteilhaft betrachtet und bräuchten daher nur bedingt in die Analyse einer Herzratenvariabilität berücksichtigt werden. Damit kann eine zusätzliche Möglichkeit zur Interpretation bzw. Analyse einer Ausprägung und Häufigkeit - sprunghafter - Änderungen einander folgender RR-Intervall-Längen erhalten werden. Eine verbesserte Darstellung bzw. Analyse in Form eines Histogramms, Streudiagramms usw. kann so ermöglicht werden.

## Patentansprüche

1. Verfahren zur Bestimmung des physischen und/oder psychischen Zustands eines Probanden anhand einer Analyse seiner Herzratenvariabilität (HRV), bei welcher Analyse wenigstens eine Häufigkeitsverteilung von in mindestens einem Untersuchungszeitraum aufgenommenen Interbeat-Intervallen (IBI) untersucht wird, **dadurch gekennzeichnet, dass** die Analyse der Herzratenvariabilität (HRV) mindestens eine Untersuchung wenigstens einer Häufigkeitsverteilung auf eine multimodale Verteilung umfasst.

2. Verfahren nach Anspruch 1, **dadurch gekennzeichnet, dass** als Interbeat-Intervalle RR-Intervalle des Herzens und/oder Abstände zwischen Pulswellen herangezogen werden.

3. Verfahren nach Anspruch 1 oder 2, **dadurch gekennzeichnet, dass** eine klassierte Häufigkeitsverteilung herangezogen wird.

4. Verfahren nach einem der Ansprüche 1 bis 3, **dadurch gekennzeichnet, dass** die Untersuchung der Häufigkeitsverteilung auf einer univariaten statistischen Analyse erfolgt.

5. Verfahren nach einem der Ansprüche 1 bis 3, **dadurch gekennzeichnet, dass** die Untersuchung der Häufigkeitsverteilung auf einer bivariaten statistischen Analyse erfolgt.

6. Verfahren Anspruch 5, **dadurch gekennzeichnet, dass** für die bivariate statistische Analyse mindestens ein Interbeat-Intervall mit seinem nachfolgenden Interbeat-Intervall herangezogen wird.

7. Verfahren nach Anspruch 5 oder 6, **dadurch gekennzeichnet, dass** die bivariate statistische Analyse eine Klassierung der Häufigkeitsverteilung in Abhängigkeit der Unterschiede zweier aufeinanderfolgender Interbeat-Intervall-Längen umfasst.

8. Verfahren nach einem der Ansprüche 1 bis 7, **dadurch gekennzeichnet, dass** die Analyse der Herzratenvariabilität den Abstand zwischen Modalwerten berücksichtigt.

9. Verfahren nach einem der Ansprüche 1 bis 8, **dadurch gekennzeichnet, dass** die Analyse der Herzratenvariabilität ein Verhältnis der Dichten der Modalverteilungen, insbesondere der Integrale dieser Dichten, berücksichtigt.

10. Verfahren nach einem der Ansprüche 1 bis 9, **dadurch gekennzeichnet, dass** bei der Bestimmung des physischen und/oder psychischen Zustands eines Probanden die Analysedaten der Herzratenvariabilität (HRV) mit Vergleichsdaten anderer Probanden in Relation gesetzt werden.

11. Verfahren nach einem der Ansprüche 1 bis 10, **dadurch gekennzeichnet, dass** die Modalwerte der Häufigkeitsverteilung unter Verwendung eines Schwellwerts bestimmt werden.

12. Verfahren nach einem der Ansprüche 1 bis 11, **dadurch gekennzeichnet, dass** die Häufigkeitsverteilungen von in wenigstens zwei Untersuchungszeiträumen aufgenommenen Interbeat-Intervallen untersucht werden, wobei in den Untersuchungszeiträumen unterschiedliche physische und/oder psychische Belastungszustände auf den Probanden wirken.

13. Verfahren nach einem der Ansprüche 1 bis 12, **dadurch gekennzeichnet, dass** mit Hilfe der Untersuchung der Häufigkeitsverteilung ein Korrekturwert für eine Zeitbereichsanalyse der Herzfrequenzvariabilität, beispielsweise für Parameter RMSSD und/oder pNN50, berechnet und dabei berücksichtigt wird.
